# EUROPÄISCHE PATENTSCHRIFT

(11) **EP 1 704 887 B1**
(45) Veröffentlichungstag und Bekanntmachung des Hinweises auf die Patenterteilung: **15.10.2008**
(21) Anmeldenummer: 05006679.4
(22) Anmeldetag: 26.03.2005
(51) Int. Cl.: A61M 5/24, A61M 5/315

(54) **Dosiervorrichtung mit Priming-Funktion**
Dosing device with priming function
Dispositif de dosage avec fonction d'amorç age

(43) Veröffentlichungstag der Anmeldung: 27.09.2006
(73) Patentinhaber: TecPharma Licensing AG, 3401 Burgdorf (CH)
(72) Erfinder: Kirchhofer, Fritz, 3454 Sumiswald (CH)

(56) Entgegenhaltungen:
- WO-A-03/020347
- WO-A-20/04064902
- US-A- 5 807 346
- US-B1- 6 241 709

## Beschreibung

Die vorliegende Erfindung betrifft eine Dosiervorrichtung, die eine halbautomatisierte Priming-Funktion durchführen kann, wobei die Dosiervorrichtung zur dosierten Verabreichung eines injizierbaren Produktes dient, gemäß dem Oberbegriff des Anspruchs 1.

Im Stand der Technik sind Vorrichtungen zur dosierten Verabreichung eines Medikaments, insbesondere Insulin, Hormone oder dgl., bekannt. Es handelt sich dabei etwa um Injektionspens, die ein Patient, etwa ein Diabetiker, bedienen kann, um sich eine gewünschte Dosis Insulin selbst zu verabreichen. Derartige Injektionspens verfügen über einen Dosierknopf, der aus einer Öffnung eines Gehäuses des Injektionspen herausragt. Zur Verabreichung einer Injektion wird die zu verabreichende Dosis mittels des Dosierknopfes ausgewählt bzw. eingestellt. Durch das Drehen des Dosierknopfes wird ein Antriebsmittel voreingestellt. Zum Injizieren wird der Dosierknopf dann aus einer ersten Endposition ein Stück weit in das Gehäuse voranbewegt, bis eine zweite Endposition erreicht wird. Dabei wird die voreingestellte Menge des zu injizierenden Produktes verabreicht. Ferner wird dabei ein Antriebsmittel voranbewegt, das auf einen Kolben einwirkt, der auf das injizierbare Produkt in einer Ampulle einwirkt, so dass das Medikament aus der Ampulle verdrängt und über eine Kanüle ausgestoßen werden kann.

Nach der Verabreichung des Medikaments bzw. des Insulins, Wachstumshormons oder dgl., ist es erforderlich, den Injektionspen für eine neuerliche Injektion vorzubereiten, indem ein Priming-Vorgang erfolgt. Hierbei wird bekanntermaßen der lichte Querschnitt der Injektionskanüle entlüftet sowie von Reststoffen befreit, indem eine bestimmte geringe Menge des zu injizierenden Produkts ausgestoßen wird, ohne eine Injektion vorzunehmen.

Dieser Priming-Vorgang bzw. dieses Priming ist für einen Patienten, der eine Injektion vorzunehmen hat, ein zusätzlicher Arbeitsgang, der auch nicht vergessen werden darf, um eine optimale Injektion nach dem Priming gewährleisten zu können. Bei einer bekannten Dosiervorrichtung bzw. einem bekannten Injektionspen wird ein Priming dadurch bewerkstelligt, dass durch Drehen eines Stellabschnittes eine Umstellung von einer Verabreichungsposition in eine Priming-Position erzielt wird. Diese Verstellung muss für eine nachfolgende Verabreichung wieder zurückgestellt werden. Dies erfordert einen zusätzlichen Aufwand und zusätzliche Aufmerksamkeit des Patienten. Gerade in Stresssituationen, wenn unbedingt eine schnelle Insulininjektion erfolgen muss, kann der Patient diese Handhabungsvorgänge unbeabsichtigt außer Acht lassen oder falsch durchführen.

Es ist dementsprechend die Aufgabe der vorliegenden Erfindung, eine Dosiervorrichtung vorzuschlagen, die eine alternative Bewerkstelligung der Priming-Funktion ermöglicht. Ferner ist es eine Zielrichtung der Erfindung, eine vereinfachte Priming-Funktion zur Verfügung zu stellen, die vorteilhafterweise in vorhandene mechanische Bestandteile der Dosiervorrichtung integriert werden kann.

Gemäß der Erfindung wird die genannte Aufgabe wenigstens teilweise durch eine Dosiervorrichtung gelöst, bei der ein Abschnitt zur Dosierung mit einer Priming-Einrichtung gekoppelt ist. Insbesondere ist das Antriebsmittel mit Eingriffselementen versehen, die in Gruppen beieinander liegen, wobei ein Dosierabstand zwischen den Gruppen einer Dosiermenge entspricht, und ein Priming-Abstand zwischen den Eingriffselementen einer Priming-Menge entspricht.

Ferner kann gemäß einer bevorzugten Ausführungsform der Dosierknopf so an das Antriebsmittel angekoppelt sein, dass der Dosierknopf bzw. das Dosierelement einmal mit einer Gruppe von Eingriffselementen und ein anderes Mal mit einem einzelnen Eingriffselement in Wechselwirkung tritt, um das eine Mal die Dosiermenge zu verabreichen und das andere Mal die Priming-Menge auszugeben. Bei dieser erfindungsgemäßen Ausgestaltung umfasst das für die dosierte Verabreichung vorhandene Antriebsmittel sogleich integral auch die Möglichkeit, die Priming-Funktion zu steuern. Es kann auf diese Weise sichergestellt werden, dass jeder Verabreichungsfunktion eine Priming-Funktion vorausgeht, respektive nachfolgt. Auch eine erste Priming-Funktion, nachdem eine neue Ampulle eingelegt worden ist, lässt sich auf diese Weise bewerkstelligen.

Natürlich kann statt eines Dosierknopfes auch ein anderes Dosierelement verwendet werden. Wesentlich ist nur, dass der Patient eine Kraft übertragen kann, die letztlich zu einer dosierten Verabreichung führt. Nachfolgend wird beispielhaft von einem Dosierknopf die Rede sein.

Vorteilhafterweise ist eine Vorschubhülse vorgesehen, die mit dem Dosierknopf verbunden ist. Die Vorschubhülse greift an dem Antriebsmittel an, um eine Antriebskraft von dem Dosierknopf auf das Antriebsmittel zu übertragen. Damit ist mit der Vorschubhülse ein Übertragungselement vorgesehen, mit dem eine Antriebskraft, die der Patient ausübt, über den Dosierknopf und die Vorschubhülse auf das Antriebsmittel transferiert werden kann, um auf einen Kolben einzuwirken, der das injizierbare Produkt aus der in der Dosiervorrichtung vorhandenen Ampulle heraustreibt.

Vorteilhafterweise umfasst eine Gruppe von Eingriffselementen mindestens zwei Eingriffselemente. Dabei wird der Abstand zwischen aufeinanderfolgenden Gruppen von Eingriffselementen verwendet, um eine maximale Verstellung zu ermöglichen, d.h., eine bestimmte vorgegebene Dosis zu verabreichen.

Der Abstand zwischen aufeinanderfolgenden Eingriffselementen einer Gruppe kann so bemessen sein, dass dieser Abstand gerade der Priming-Menge entspricht, die zur Ausübung der Priming-Funktion auszustoßen ist.

Würde eine Person, die die erfindungsgemäße Dosiervorrichtung bedient, eine Verabreichungsfunktion durchführen wollen, würde die Person, etwa ein Patient, an dem Dosierknopf ziehen, wodurch dieser die Vorschubhülse axial entgegengesetzt zur Ausstoßrichtung der vorhandenen Injektionsnadel bewegt. Die Vorschubhülse wird über einen Dosierabstand bewegt, so dass eine Eingriffsfalle an einem wenigstens im Querschnitt zangenartigen Abschnitt der Vorschubhülse in Eingriff mit einem Eingriffselement einer nachfolgenden Gruppe von Eingriffselementen des Antriebsmittels gelangen kann. Anschließend könnte der Patient den Dosierknopf in Ausstoßrichtung der Injektionsnadel bewegen, wodurch der Dosierknopf die Vorschubhülse axial nach vorne bewegt und dabei das Antriebsmittel, etwa eine Gewindestange, eine Zahnstange, oder dgl., vorantreibt. Das Antriebsmittel wirkt auf den Kolben ein und veranlasst den Ausstoß einer vorbestimmten Menge an injizierbarem Produkt, etwa Insulin. Anschließend kann der Patient wiederum zur Vorbereitung einer nächsten Injektion wiederum am Dosierknopf ziehen, wodurch dann die Falle an der Vorschubhülse außer Eingriff mit dem Eingriffselement der Gruppe kommt und zu einem nachfolgenden Eingriffselement derselben Gruppe bewegt wird. Sollte der Patient den Dosierknopf vollkommen herausziehen, über den Abstand, der einem Dosierabstand entspricht, so würde die Falle an der Vorschubhülse über das Antriebsmittel gleiten, und zwar über einen Abstand, über den kein Eingriffselement vorgesehen ist, und die Falle an der Vorschubhülse könnte mit den Eingriffselementen der nachfolgenden Gruppe nicht in Eingriff gelangen, weil der zusätzlich erforderliche, zu überbrückende Abstand von der Vorschubhülse aufgrund der beschränkten Strecke, die der Dosierknopf überwinden kann, nicht überwunden werden kann. Bei einer nachfolgenden Betätigung des Dosierknopfes wird die Vorschubhülse ohne in Eingriff mit einem Eingriffselement gelangen zu können, wieder voranbewegt werden. Erst wenn die Falle der Vorschubhülse mit dem letzten Eingriffselement der vorangehenden Gruppe in Eingriff gelangt ist, kann das Antriebsmittel wieder voranbewegt werden und entsprechend dem geringen Abstand zwischen den Eingriffselementen einer Gruppe wird lediglich eine Priming-Menge an injizierbarem Produkt ausgestoßen.

Nachdem dieser Vorgang abgeschlossen ist, kann der Patient dann den Dosierknopf wieder nach hinten ziehen, wodurch die Falle an der Vorschubhülse wieder außer Eingriff mit dem letzten Eingriffselement gelangt, über das Antriebsmittel gleitet, solange kein Eingriffselement einer nachfolgenden Gruppe vorhanden ist, um dann, wenn der Dosierknopf die maximal mögliche Strecke herausgezogen worden ist, in Eingriff mit einem Eingriffselement einer nachfolgenden Gruppe zu gelangen. Diese maximale Strecke entspricht dann wiederum einer maximalen Vortriebsbewegung, die im Prinzip eine maximale Dosierung auszulösen vermag. Demnach sollte eine Gruppe von Eingriffselementen wenigstens zwei Eingriffselemente aufweisen, wobei der Abstand zwischen den beiden Eingriffselementen einer Gruppe der Priming-Menge entsprechen sollte und der Abstand zwischen zwei Gruppen einer maximalen Dosierung entsprechen sollte. Von einer maximalen Dosierung abweichende Dosierungsmengen können anderweitig eingestellt werden, beispielsweise über einen einstellbaren Vortriebsweg für das Antriebsmittel und/oder die Vorschubhülse. D.h., während der Dosierknopf in Verbindung mit der Vorschubhülse immer die gleiche Strecke zurückgezogen werden kann, kann das Antriebsmittel bzw. kann die Vorschubhülse einen einstellbaren Weg vorangetrieben werden, um unterschiedliche Mengen des injizierbaren Produktes verabreichen zu können.

Eine Gruppe von Eingriffselementen wird dabei vorteilhafterweise dadurch bestimmt, dass zwischen Gruppen von Eingriffselementen ein größerer Abstand ist als zwischen den Eingriffselementen einer Gruppe. Diese Regel sollte prinzipiell zutreffen, da eine Priming-Menge regelmäßig kleiner sein wird, als eine Dosierungsmenge.

Um dem Patienten die Handhabung weiter erleichtern zu können, kann die Dosiervorrichtung gemäß der Erfindung mit einer Priming-Anzeige versehen sein. Diese wird ein Signal zu erkennen geben, das eine Betätigung des Antriebsmittels und/oder des Dosierknopfes eine Priming-Funktionsbereitschaft bewirkt hat. Der Patient muss auf diese Weise den aktuellen Funktionszustand der erfindungsgemäßen Vorrichtung nicht in Erinnerung rufen, was die Funktionssicherheit weiter erhöht.

Vorteilhafterweise ist ferner eine Führungseinrichtung, etwa eine Führungshülse vorgesehen, die ebenfalls mit den Eingriffselementen des Antriebsmittels in Eingriff zu gelangen vermag, um das Antriebsmittel in einer Position halten zu können, während die Vorschubeinrichtung über den Dosierknopf zurückgezogen wird. Über die Eingriffsposition der Führungseinrichtung und die der Vorschubhülse, d.h., den Abstand dazwischen, ließe sich eine variable Dosierung bewerkstelligen. Dabei würde auch ein Anschlag an der Vorschubhülse passend zu verstellen sein, so dass zwar der Dosierknopf regelmäßig über die gleiche Strecke zurückgezogen werden kann, aber regelmäßig über die gleiche Strecke zurückgezogen werden kann, aber die Vortriebsbewegung verstellbar beschränkt ist, passend zum Abstand der Eingriffspositionen.

Gemäß einer vorteilhaften Ausführungsform der Erfindung weist das Antriebsmittel zwischen wenigstens einigen der Gruppen von Eingriffselementen gleiche Abstände auf. Mittels dieser gleichen Abstände zwischen den Gruppen ist es möglich, eine einzelne Dosiermenge eines injizierbaren Produktes fest einzustellen. Die erfindungsgemäße Dosiervorrichtung wird auch als Einmal-Pen eingesetzt, d.h., es braucht dann keine zusätzliche Ampulle vorhanden sein, sondern es ist auch möglich, das Produkt unmittelbar ohne austauschbare Ampulle in einer Aufnahme des Gehäuses unterzubringen.

Gemäß einer bevorzugten Ausführungsform ist das Antriebsmittel zwischen den Gruppen glatt ausgebildet, bzw. zwischen den Gruppen weist das Antriebsmittel keine zu den Gruppe gehörigen Eingriffselemente oder Strukturen auf.

Nachfolgend wird die vorliegende Erfindung anhand einer bevorzugten Ausführungsform unter Bezugnahme auf die beigefügten Figuren näher erörtert. Dabei werden weitere Vorteile, Zielsetzungen und Merkmale gemäß der Erfindung offenbart. In den Darstellungen zeigen:
- Fig. 1: einen axialen Längsschnitt durch eine Vorrichtung der Erfindung;
- Fig. 2: einen axialen Längsschnitt durch einen gemäß der Erfindung modifizierten Antriebsbereich der Ausführungsform gemäß Fig. 1 in einer Verabreichungsstellung;
- Fig. 3: eine Fig. 2 entsprechende Darstellung, wobei eine Priming-Funktion ausgeführt wird;
- Fig. 4: eine den Fig. 2 und 3 entsprechende Darstellung, wobei eine Dosierung durchgeführt worden ist und eine Priming-Funktion ansteht; und
- Fig. 5a bis 5c: einen Schnitt durch die Vorrichtung gemäß Fig. 1 im Bereich der Priming-Anzeige in verschiedenen Positionen.

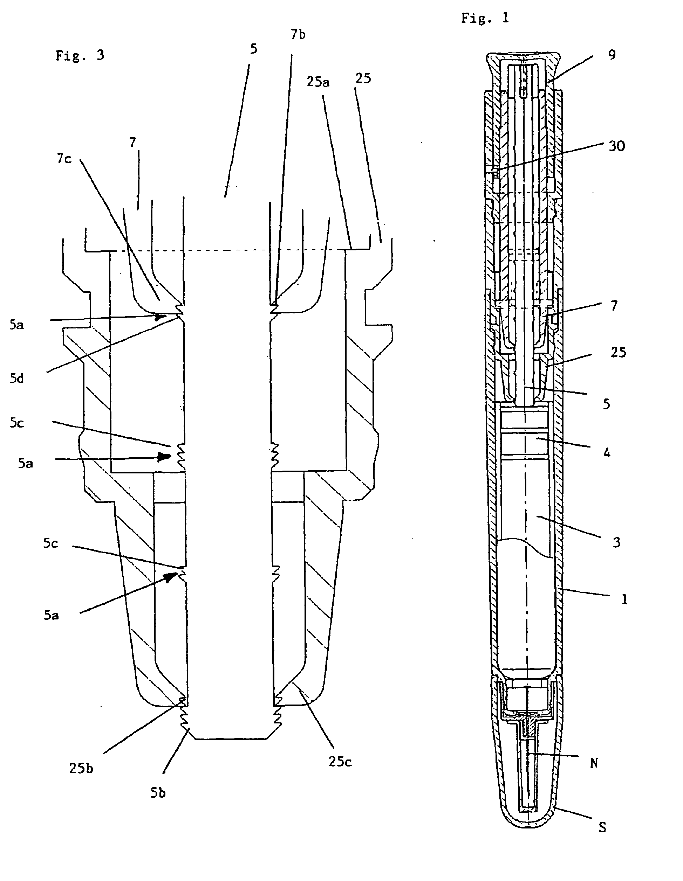

In der Fig. 1 ist eine Vorrichtung mit Merkmalen gemäß der Erfindung dargestellt, die ein Gehäuse 1 umfasst, aus dem stirnseitig eine Injektionsnadel bzw. Kanüle N heraussteht, die von einer Schutzkappe S umgeben wird. Eine weitere Umhüllung für die Kanüle der Injektionsnadel N kann vorgesehen sein, wird jedoch in der Regel bei Inbetriebnahme entfernt werden und erst bei der Anbringung einer neuen Injektionsnadel N aus Gründen der Sterilität angeordnet werden.

In dem Gehäuse 1 ist weiterhin eine Ampulle 3 vorgesehen, die mit einem Kolben 4 ausgestattet ist, der innerhalb der Ampulle vorangetrieben werden kann, um ein injizierbares Produkt ausstoßen zu können. In Axialrichtung des zylinderförmigen Gehäuses 1 schließt ein Antriebsmittel 5, etwa eine Zahnstange, eine Gewindestange oder dgl. an den Kolben 4 an. Das Antriebsmittel wird innerhalb einer Führungseinrichtung, insbesondere einer Führungshülse 25, und einer Vorschubeinrichtung, insbesondere einer Vorschubhülse 7 in Axialrichtung voran in Richtung der Injektionsnadel N oder zurück in Richtung eines Dosierknopfes 9 bewegt.

Der Dosierknopf ist an der Vorschubeinrichtung bzw. Vorschubhülse 7 festgelegt. Der Dosierknopf 9 weist eine Anzeige 30 auf, die anzeigen soll, wenn eine Priming-Funktion durchführbar ist.

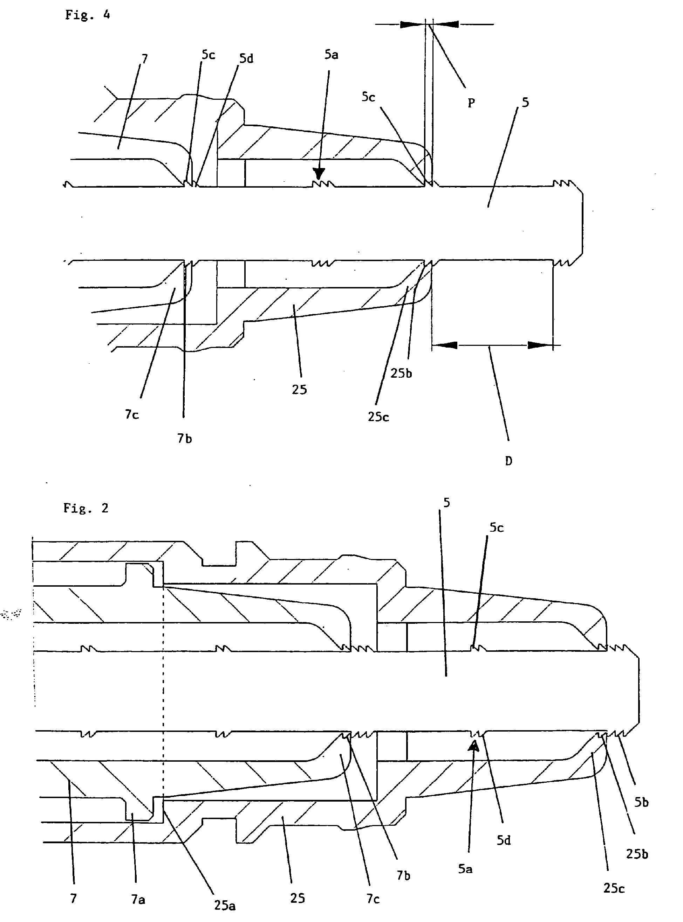

Die gemäß der Erfindung hervorzuhebenden Bestandteile der erfindungsgemäßen Dosiervorrichtung werden nachfolgend gesondert anhand der Fig. 2 bis 5 beschrieben. Dabei ist festzuhalten, dass in den Figuren für gleiche oder funktionsgleiche Bestandteile jeweils gleiche Bezugszeichen verwendet werden, so dass sich eine mehrfache Beschreibung der gleichen Bestandteile erübrigt.

In der Fig. 2 sind zunächst das Antriebsmittel, hier eine Zahnstange 5, die Vorschubhülse 7 und die Führungshülse 25 generell in einer Schnittdarstellung wiedergegeben worden.

Die Zahnstange 5 weist mehrere Gruppen 5a von Eingriffselementen 5b, 5c, 5d auf, die ihrer Funktion entsprechend bestimmte Abstände untereinander aufweisen, was insbesondere in Fig. 4 verdeutlicht wird. Eine vordere Gruppe 5a umfasst drei Zähne, die angeordnet sind, um nach einer Platzierung einer neuen Ampulle ein erstes Entlüften bzw. ein erstes Priming zu ermöglichen. Korrespondierend hierzu ist eine übernächste Gruppe 5a von Zähnen ebenfalls mit drei Zähnen ausgebildet. Die zu der vorderen Gruppe nachfolgende Gruppe 5a besteht aus zwei Zähnen und die der nachfolgenden Gruppe 5a mit drei Zähnen nachfolgende Gruppe umfasst wiederum zwei Zähne, die einerseits einer Verabreichung einer bestimmten Dosis - vorderer Zahn 5d - und einer Priming-Funktion genügen. Sämtliche nachfolgenden Gruppen 5a (hier nicht erkennbar) können dann ebenfalls lediglich zwei Zähne aufweisen, um sowohl die dosierte Verabreichung als auch die Priming-Funktion zu bewerkstelligen.

Die Vorschubhülse 7 umfasst zwei zahnartige Backen 7c, die jeweilige Eingriffsfallen 7b aufweisen, die mit den Eingriffselementen bzw. Zähnen der Gruppen 5a von Eingriffselementen in Eingriff bringbar sind. Die Zähne 5b bis 5d sind so geformt, dass bei einer Voranbewegung der Zahnstange 5 die Backen 7c auseinandergespreizt werden können, wobei die Zähne in Vorschubrichtung der Zahnstange 5 relativ zur Zylinderachse des zylinderförmigen Gehäuses 1 (siehe Fig. 1) der erfindungsgemäßen Vorrichtung eine Flanke aufweisen, die einen eher spitzen Winkel mit der Zylinderachse des Gehäuses 1 (siehe Fig. 1) einschließen, der beispielsweise in einem Bereich von etwa 30 bis 60° liegen kann. Durch diese Ausgestaltung ist es möglich, die Zähne 5b - 5d außer Eingriff mit der Falle 7b der Vorschubhülse gelangen zu lassen.

Die in Vorschubrichtung jeweils hintere Flanke eines jeden Zahnes der Zahnstange 5 ist dagegen sehr steil ausgebildet, so dass entgegen der Vorschubrichtung der Zahnstange 5 die Stirnseite der zangenartigen Backe 7c, die ebenfalls senkrecht zur Zylinderachse des Gehäuses ausgerichtet ist, entgegen der Vorschubrichtung einen großen Widerstand entgegensetzt, so dass ein Verschieben der Zahnstange 5 entgegen der Vorschubrichtung nicht möglich ist.

Die Führungseinrichtung bzw. Führungshülse 25 ist ähnlich mit zwei zangenartigen Backen 25c ausgebildet, wie die Vorschubhülse 7. Die zangenartigen Backen 25c weisen ebenfalls Fallen 25b auf, die mit den Zähnen 5b - 5d in Eingriff bringbar sind, um die Zahnstange 5 in einer Position zu halten, während über den Dosierknopf 9 an der Vorschubhülse 7 gezogen wird, um die Vorschubhülse 7 außer Eingriff mit dem aktuellen Zahn zu bringen, um die erfindungsgemäße Vorrichtung in einen anderen Betriebszustand zu versetzen.

Die Führungshülse 25 weist einen Anschlag 25a auf, an den ein korrespondierender Anschlag 7a (siehe Figur 2) der Vorschubhülse anschlägt, um in Vorschubrichtung die Vorschubbewegung der Vorschubhülse gegenüber der Führungshülse 25 zu begrenzen, wodurch korrespondierend natürlich auch eine Vorschubbewegung der Zahnstange 5 begrenzt wird.

Eine Abfolge von aufeinanderfolgenden Verabreichungs- und Priming-Vorgängen erfolgt, indem zunächst eine neue Ampulle 3 in das Gehäuse 1 der erfindungsgemäßen Vorrichtung eingefügt wird, um dann eine erste Entlüftung der Injektionsnadel N vorzunehmen. In dieser Situation tritt die Falle 7b der zangenartigen Backe 7b in Eingriff mit einem Eingriffselement der bereits in der vorgeschobenen Stellung wiedergegebenen Gruppen 5a mit drei Zähnen und bewegt die Zahnstange 5 in axialer Richtung nach vorne sobald der Dosierknopf in Richtung der Kanüle N (siehe Fig. 1) gedrückt wird. Der dritte Zahn von vorne der vorderen Gruppe mit drei Zähnen gelangt außer Eingriff mit den Fallen 25b an den vorderen zangenartigen Backen 25c der Führungshülse 25 und hakt hinter die rückwärtige Flanke des hintersten Zahnes der vordersten Gruppe von Zähnen hinter. Die Stirnseite der Zahnstange 5 drückt so auf den Kolben 4 und ein erstes Priming erfolgt und versetzt die erfindungsgemäße Dosiervorrichtung in einen Bereitschaftszustand. Anschließend wird an dem Dosierknopf 9 gezogen. Der Dosierknopf 9 ist fest mit der Vorschubhülse 7 verbunden und durch das Zurückziehen des Dosierknopfes 9 gerät die Falle 7b am vorderen Ende der Vorschubhülse 7 außer Eingriff zu dem Zahn, der zuvor im Eingriff gewesen ist.

Wird ein Dosiervorgang durchgeführt, werden die zangenartigen Backen 7c über einen zahnlosen Schaftabschnitt der Zahnstege 5 zwischen den Gruppen von Zähnen rutschen, um dann mit einem nachfolgenden Zahn einer nachfolgenden Gruppe in Eingriff zu gelangen. In dieser Situation ist dann der Dosierknopf vollkommen herausgezogen und durch Drücken des Dosierknopfes 9 in Richtung der Injektionsnadel N kann der Kolben 4 innerhalb der Ampulle 3 vorangetrieben werden um die Verabreichung einer vorgegebenen Dosis des injizierbaren Produktes zu veranlassen.

Wird bei der nächsten Betätigung der Dosierknopf 9 wieder zurückgezogen so wird die Vorschubhülse 7 mit zurückbewegt und rutscht über die schräge Flanke des nachfolgenden Zahnes der Zahnstange 5, und zwar einem Zahn 5c einer entsprechenden Gruppe 5a von Zähnen. Wird an dem Dosierknopf weiter gezogen, rutscht dieser über den zahnlosen Bereich der Zahnstange 5, kann aber im Rahmen seiner Reichweite einen nachfolgenden Zahn einer nachfolgenden Gruppe nicht erreichen. Wenn der Dosierknopf anschließend niedergedrückt wird, rutschen die zangenartigen Backen 7c wieder zurück über den zahnlosen Bereich der Zahnstange 5 und haken dann hinter eine steile Flanke eines Zahnes hinter, um anschließend eine Priming-Funktion ausüben zu können, wenn lediglich ein Vorschub über eine geringe Entfernung erfolgt.

Die zangenartigen Backen 25c, 7c sind insoweit radial flexibel und werden radial ausgelenkt, wenn am Dosierknopf und damit an der Vortriebshülse gezogen wird, wobei die Falle 7b, 25b über den jeweils zugeordneten Zahn der Zahnstange 5 rutscht. Anschließend schnellen die jeweiligen zangenartigen Backen in ihre radiale Ausgangslage zurück.

Anschließend wiederholen sich die voranstehend beschriebenen Aktionen, wobei natürlich lediglich das allererste Priming nach dem Einsetzen einer neuen Ampulle entfällt.

In Fig. 4 ist explizit der Dosierabstand D zwischen aufeinanderfolgenden Gruppen von Zähnen dargestellt worden. Genauso ist der Priming-Abstand P zwischen aufeinanderfolgenden Zähnen einer Gruppe von Zähnen wiedergegeben. Es ist gut zu erkennen, dass der Dosierabstand wesentlich größer als der Priming-Abstand ist.

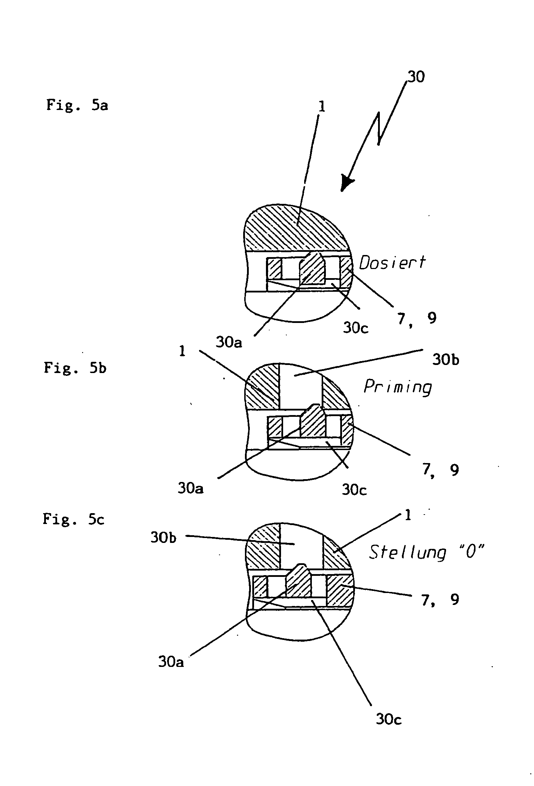

Die Fig. 5a bis 5c zeigen die Priming-Anzeige 30 in einer Detaildarstellung. Die erfindungsgemäße Dosiervorrichtung im dosierten Zustand gemäß Fig. 5a ist ein Priming-Anzeigenocken 30a in Anlage zu der Wandung des Gehäuses 1 der erfindungsgemäßen Dosiervorrichtung und folglich von außen nicht erkennbar. Eine Blattfeder 30c drückt den Anzeigenocken 30a in Richtung der Wandung des Gehäuses 1. Die Blattfeder 30c ist an einem unteren Abschnitt des Dosierknopfes 9 bzw. der Vorschubhülse 7 angebracht. Wird nun an dem Dosierknopf 9 gezogen, kann der Anzeigenocken 30a in einen Anzeigeschlitz 30b in der Wandung des Gehäuses 1 rutschen und ist dann von außen erkennbar. Diese Stellung soll anzeigen, das die erfindungsgemäße Dosiervorrichtung für einen Priming-Vorgang bereit ist und ein Priming durchzuführen ist.

In der Fig. 5c ist eine Stellung "Null" dargestellt, in der der Anzeigenocken 30a eine Neutralstellung der erfindungsgemäßen Dosiervorrichtung zu erkennen gibt.

Damit wird gemäß der Erfindung eine Dosiervorrichtung zur Verfügung gestellt, die den Priming-Vorgang für den Benutzer, d.h., den Patienten, erleichtert. Der Benutzer ist lediglich darauf angewiesen, die entsprechenden Funktionen aufeinanderfolgend auszuführen und, wenn ein Priming durchzuführen ist, keine Injektion vorzunehmen und umgekehrt.

## Patentansprüche

1. Dosiervorrichtung zur dosierten Verabreichung eines injizierbaren Produkts, wobei die Vorrichtung umfasst:
a) ein Gehäuse (1) mit einer Aufnahme (3) für das Produkt,
b) ein Fördermittel (4) für eine Dosis des zu verabreichenden Produkts,
c) ein Antriebsmittel (5, 7) für das Fördermittel (4),
d) ein Dosierelement (9), das mit dem Antriebsmittel (5, 7) gekoppelt ist, das in dem Gehäuse (1) verschiebbar gelagert ist, und das Eingriffselemente aufweist,
**gekennzeichnet durch** die folgenden Merkmale:
e) die Eingriffselemente (5b, 5c, 5d) liegen, in beabstandeten Gruppen (5a) beieinander, wobei ein Dosier-Abstand (D) zwischen den beabstandeten Gruppen einer Dosiermenge des injizierbaren Produktes entspricht und ein Priming-Abstand (P) zwischen den Eingriffselementen (5b, 5c, 5d) einer Gruppe einer Priming-Menge entspricht.

2. Dosiervorrichtung nach Anspruch 1, **dadurch gekennzeichnet, dass** das Dosierelement, insbesondere ein Dosierknopf (9), so über eine Verschiebeeinrichtung (7) an das Antriebsmittel (5) angekoppelt ist, dass das Dosierelement einmal mit einer beabstandeten Gruppe von Eingriffselementen und ein anderes Mal mit einem Eingriffselement (5b, 5c, 5d) einer Gruppe in Wechselwirkung tritt, um das eine Mal die Dosiermenge zu verabreichen und das andere Mal die Priming-Menge auszugeben.

3. Dosiervorrichtung nach Anspruch 1, **dadurch gekennzeichnet, dass** eine Verschiebeeinrichtung (7) vorgesehen ist, die mit dem Dosierelement (9) verbunden ist und die an dem Antriebsmittel (5) angreift, um eine Antriebskraft von dem Dosierknopf (9) auf das Antriebsmittel (5) zu übertragen.

4. Dosiervorrichtung nach einem der Ansprüche 1 bis 3, **dadurch gekennzeichnet, dass** mindestens eine der beabstandeten Gruppen (5a) von Eingriffselementen (5b, 5c, 5d) aus zwei Eingriffselementen besteht.

5. Dosiervorrichtung nach einem der Ansprüche 1 bis 4, **dadurch gekennzeichnet, dass** die Gruppen von Eingriffselementen **dadurch** bestimmt sind, dass zwischen Gruppen (5a) ein größerer Abstand vorgesehen ist, als zwischen den Eingriffselementen (5b, 5c, 5d) einer Gruppe (5a).

6. Dosiervorrichtung nach einem der Ansprüche 1 bis 5, **dadurch gekennzeichnet, dass** eine Priming-Anzeige (30) vorgesehen ist, die ein Signal abgibt, das eine Betätigung des Antriebsmittels und/oder des Dosierknopfes eine Priming-Funktionsbereitschaft bewirkt hat.

7. Dosiervorrichtung nach Anspruch 6, **dadurch gekennzeichnet, dass** die Priming-Anzeige (30) durch die Verschiebeeinrichtung, insbesondere eine Vorschubhülse (7) auslösbar ist, indem die Vorschubhülse mechanisch an die Priming-Anzeige (30) angekoppelt ist und diese bewegt.

8. Dosiervorrichtung nach einem der Ansprüche 1 bis 7, **dadurch gekennzeichnet, dass** eine Führungseinrichtung (25) vorgesehen ist, die das Antriebsmittel (5) in einer Position hält, in die das Antriebsmittel durch ein Vorschieben versetzt worden ist.

9. Dosiervorrichtung nach einem der Ansprüche 1 bis 8, **dadurch gekennzeichnet, dass** zwischen wenigstens einigen der Gruppen (5) gleiche Abstände eingerichtet sind, um gleiche Dosiermengen verabreichen zu können.

10. Dosiervorrichtung nach einem der Ansprüche 1 bis 9, **dadurch gekennzeichnet, dass** das Antriebsmittel (5) zwischen den Gruppen (5a) glatt ist bzw. keine zu den Gruppen gehörigen Eingriffselemente hat.

## Claims

1. Dosing device for administering an injectable product, whereby the device comprises:
a) a housing (1) with a receptacle (3) for the product,
b) a propulsion means (4) for a dose of the product being administered,
c) an actuating system (5, 7) for the propulsion means (4),
d) a dosing element (9) which is linked to the actuating system (5, 7) which is accommodated in the housing (1) such that it can be moved and which has engagement elements,
**characterised by** the following features:
e) the engagement elements (5b, 5c, 5d) are arranged in groups (5a) separated from each other whereby a dosing length (D) between the separated groups corresponds to a dosing quantity of the injectable product, and a priming length (P) between the engagement elements (5b, 5c, 5d) of a group corresponds to a priming quantity.

2. Dosing device in accordance with claim 1, **characterised in that** the dosing element, in particular a dosing button (9), is linked via a displacement device (7) to the actuator (5) such that the dosing element interacts at one time with separated group of engagement elements and, at another time, with an engagement element (5b, 5c, 5d) of a group so that, at the one time, the dosing quantity is administered and, at the other time, the priming function is operated.

3. Dosing device in accordance with claim 1, **characterised in that** a displacement device (7) is provided which is linked to the dosing element (9) and which engages with the actuator (5) to transfer an actuating force from the dosing button (9) to the actuator (5).

4. Actuator in accordance with one of claims 1 to 3, **characterised in that** at least one of the separated groups (5a) of engagement elements (5b, 5c, 5d) comprises two engagement elements.

5. Dosing device in accordance with one of claims 1 to 4, **characterised in that** the groups of engagement elements are specified in such a way that there is a larger gap between groups (5a) than between the engagement elements (5b, 5c, 5d) of a group (5a).

6. Dosing device in accordance with one of claims 1 to 5, **characterised in that** a priming indicator (30) is provided which provides a signal confirming that operation of the actuating system and/or the dosing button has set the priming function.

7. Dosing device in accordance with claim 6, **characterised in that** the priming indicator (30) can be triggered by the displacement device, in particular a sleeve-shaped pusher (7), whereby the sleeve-shaped pusher (7) is connected mechanically to the priming indicator (30) and moves it.

8. Dosing device in accordance with one of claims 1 to 7, **characterised in that** a guide device (25) is provided which retains the actuator (5) in a position to which the actuator has been moved by pushing.

9. Dosing device in accordance with one of claims 1 to 8, **characterised in that** equal gaps are arranged between at least some of the groups (5) in order to be capable of administering equal doses.

10. Dosing device in accordance with one of claims 1 to 9, **characterised in that** the actuator (5) is smooth between groups (5a), or has no engagement elements associated with the groups.

## Revendications

1. Dispositif de dosage pour l'administration dosée d'un produit injectable, le dispositif comprenant :
a) un boîtier (1) muni d'un réceptacle (3) pour le produit,
b) un moyen de transport (4) pour une dose du produit à administrer,
c) un moyen d'entraînement (5, 7) pour le moyen de transport (4),
d) un élément de dosage (9), qui est couplé au moyen d'entraînement (5, 7), lequel est logé avec possibilité de translation dans le boîtier (1), et qui présente des éléments d'engrènement,
**caractérisé par** les caractéristiques suivantes :
e) les éléments d'engrènement (5b, 5c, 5d) sont proches les uns des autres dans des groupes (5a) mutuellement distants, un écart de dosage (D) entre les groupes mutuellement distants correspondant à une quantité de dosage du produit injectable, et un écart d'amorçage (P) entre les éléments d'engrènement (5b, 5c, 5d) d'un groupe correspondant à une quantité d'amorçage.

2. Dispositif de dosage selon la revendication 1, **caractérisé en ce que** l'élément de dosage, en particulier un bouton de dosage (9), est couplé au moyen d'entraînement (5) par l'intermédiaire d'un système de déplacement (7) de manière à ce que l'élément de dosage entre en interaction tantôt avec un groupe distant constitué d'éléments d'engrènement et tantôt avec un élément d'engrènement (5b, 5c, 5d) d'un groupe, afin tantôt d'administrer la quantité de dosage et tantôt de distribuer la quantité d'amorçage.

3. Dispositif de dosage selon la revendication 1, **caractérisé en ce qu'**il est prévu un système de déplacement (7), qui est relié à l'élément de dosage (9) et qui agit sur le moyen d'entraînement (5) afin de transmettre une force d'entraînement du bouton de dosage (9) au moyen d'entraînement (5).

4. Dispositif de dosage selon l'une quelconque des revendications 1 à 3, **caractérisé en ce qu'**au moins un des groupes (5a) mutuellement distants constitués d'éléments d'engrènement (5b, 5c, 5d) se compose de deux éléments d'engrènement.

5. Dispositif de dosage selon l'une quelconque des revendications 1 à 4, **caractérisé en ce que** les groupes d'élément d'engrènement sont définis par le fait qu'il est prévu une plus grande distance entre les groupes (5a) qu'entre les éléments d'engrènement (5b, 5c, 5d) d'un groupe (5a).

6. Dispositif de dosage selon l'une quelconque des revendications 1 à 5, **caractérisé en ce qu'**il est prévu un indicateur d'amorçage (30), qui émet un signal indiquant qu'un actionnement du moyen d'entraînement et/ou du bouton de dosage a entraîné une disponibilité de fonctionnement pour l'amorçage.

7. Dispositif de dosage selon la revendication 6, **caractérisé en ce que** l'indicateur d'amorçage (30) peut être déclenché par le système de déplacement, en particulier par une douille d'avance (7), par le fait que la douille d'avance est couplée mécaniquement à l'indicateur d'amorçage (30) et le déplace.

8. Dispositif de dosage selon l'une quelconque des revendications 1 à 7, **caractérisé en ce qu'**il est prévu un système de guidage (25), qui maintient le moyen d'entraînement (5) dans une position dans laquelle le moyen d'entraînement a été déplacé par un mouvement vers l'avant.

9. Dispositif de dosage selon l'une quelconque des revendications 1 à 8, **caractérisé en ce que** l'on a prévu les mêmes distances entre au moins quelques groupes (5a) afin de pouvoir administrer les mêmes quantités de dosage.

10. Dispositif de dosage selon l'une quelconque des revendications 1 à 9, **caractérisé en ce que** le moyen d'entraînement (5) entre les groupes (5a) est lisse et/ou ne possède aucun élément d'engrènement faisant partie des groupes.
